(19) Europäisches Patentamt / European Patent Office / Office européen des brevets

(11) **EP 3 532 805 B1**

(12) **FASCICULE DE BREVET EUROPEEN**

(45) Date de publication et mention de la délivrance du brevet:
**19.11.2025   Bulletin 2025/47**

(21) Numéro de dépôt: **17797404.5**

(22) Date de dépôt: **24.10.2017**

(51) Classification Internationale des Brevets (IPC):
*D02G 3/44* *(2006.01)*      *G01D 21/00* *(2006.01)*
*A61B 5/00* *(2006.01)*      *A61B 5/0531* *(2021.01)*
*G01K 7/16* *(2006.01)*      *G01L 1/00* *(2006.01)*

(52) Classification Coopérative des Brevets (CPC):
**G01D 21/00; A61B 5/0531; A61B 5/6804;
D02G 3/441; G01K 7/16; G01L 1/005;**
A61B 2560/0468

(86) Numéro de dépôt international:
**PCT/FR2017/052923**

(87) Numéro de publication internationale:
**WO 2018/078270 (03.05.2018 Gazette 2018/18)**

(54) **FEUILLE TEXTILE/PLASTIQUE CONNECTÉE**

VERBUNDENE KUNSTSTOFF-/TEXTILFOLIE

CONNECTED PLASTIC/TEXTILE SHEET

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité:   **27.10.2016  FR 1660455**

(43) Date de publication de la demande:
**04.09.2019   Bulletin 2019/36**

(73) Titulaire: **Saint-Gobain Adfors
92400 Courbevoie (FR)**

(72) Inventeurs:
• **BOUCHARD, Jonas**
  **75017 Paris (FR)**
• **BLONDEAU, François**
  **77100 Meaux (FR)**
• **KASMER, Mehmet**
  **59150 Wattrelos (FR)**

(74) Mandataire: **Saint-Gobain Recherche
41 Quai Lucien Lefranc
93300 Aubervilliers (FR)**

(56) Documents cités:
WO-A1-01/75778          BE-A5- 1 017 472
US-A- 4 400 684          US-A- 4 794 365
US-A1- 2006 254 366      US-A1- 2008 170 982

• LEE JAEHONG ET AL: "Recent Advances in 1D Stretchable Electrodes and Devices for Textile and Wearable Electronics: Materials, Fabrications, and Applications", ADVANCED MATERIALS, vol. 32, no. 5, 1 January 2019 (2019-01-01), DE, pages 1 - 28, XP055974696, ISSN: 0935-9648
• CHEN SHUAI ET AL: "Polymer-Enhanced Highly Stretchable Conductive Fiber Strain Sensor Used for Electronic Data Gloves", ADVANCED MATERIALS TECHNOLOGIES, vol. 1, no. 7, 22 August 2016 (2016-08-22), DE, pages 1600136, XP055974746, ISSN: 2365-709X, DOI: 10.1002/ admt.201600136

## Description

La présente invention est relative au domaine des revêtements intelligents. Il s'agit en effet d'un textile ou d'une feuille plastique technique connectée et utilisée pour détecter et localiser de façon surfacique des contraintes mécaniques (système antieffraction) ou des problèmes d'infiltration d'eau sur ou dans des substrats tels que les plaques de plâtre, mortier, béton, dalle, brique, bois, carrelage, tuyau, la laine ou mousse d'isolation, ou encore des géotextiles.

## ART ANTÉRIEUR

Il existe actuellement une multitude de technologies de capteurs textiles proposant diverses natures de détection pour des applications très variées. Les capteurs textiles font partie de ce que nous appelons communément les textiles intelligents. Les textiles intelligents ont anciennement été définis comme des textiles capables de détecter et réagir à des phénomènes et conditions environnementaux tels qu'une sollicitation mécanique, un changement thermique, une réaction chimique ou une stimulation électrique.

Il est par exemple connu d'avoir un vêtement utilisant un textile connecté pour opérer une commande, par exemple pour commander un lecteur audio. Un tel tissu connecté utilise un capteur linéaire comportant des fibres textiles électriquement conductrices et des fibres textiles isolantes. Le capteur comprend au moins deux éléments conducteurs ayant des fibres textiles électriquement conductrices. Les fibres textiles isolantes sont agencées pour séparer les deux éléments électriquement conducteurs en l'absence de pression appliquée audit capteur. Lors d'une pression sur ledit capteur, une conduction électrique apparait entre les deux éléments conducteurs permettant la commande.

Un premier inconvénient des solutions existantes de capteurs textiles est qu'elles sont conçues pour des surfaces relativement petites (chaussures, vêtements, siège) par l'intermédiaire des matériaux piézorésistifs ou pour des surfaces très grandes (route, pont, aéroport) via les fibres optiques.

Un second inconvénient est que ces solutions ne sont pas multifonctionnelles. En effet, chaque solution ne vise à détecter qu'un seul paramètre à la fois (eau, contraintes mécaniques et/ou même élévation de la température).

Les documents suivants sont connus :

- US 4 400 684 décrit un capteur de température agencé sur un substrat ayant une faible conductivité thermique et une faible capacité thermique, le capteur étant du type thermocouple à réponse rapide ;
- US 2006/254366 décrit un capteur pour surveiller une structure, ledit capteur comprenant un réseau de voies électriques interconnectées, dans lequel une propriété électrique des voies (de préférence au moins l'une de l'impédance, la capacité, l'inductance et la résistance) est agencée pour répondre à une modification d'une propriété physique prédéterminée de la structure,
- WO 01/75778 décrit un capteur comprenant un tissage comprenant des fils conducteurs, chaque fil conducteur se présentant sous la forme d'un noyau associé à des brins ;
- US 2008/170982 décrit des procédés de fabrication de fils de nanofibres. Dans certains modes de réalisation, les fils de nanotubes comprennent des nanotubes de carbone ;
- US 4 794 365 décrit un capteur de pression comprenant un conducteur électrique interne au moins partiellement entouré par une couche partiellement résistive comprenant soit des fibres, soit des particules en l'absence substantielle d'un liant solide continu ;
- BE 1 017 472 décrit l'utilisation d'un fil mélangé spécial comme senseur de conduction longitudinale dans un textile détecteur de pression. Ce fil spécial est un mélange de fibres conductrices et non conductrices ;
- « Recent Advances in 1D Stretchable Electrodes and Devices for Textile and Wearable Electronics: Materials, Fabrications, and Applications », Lee Jaehong ET AL, Advanced Materials, vol. 32, no. 5, 1 janvier 2019, pages 1-28, est un résumé d'avancées technologiques dans le domaine des dispositifs électroniques extensibles 1D en termes de matériaux conducteurs et de processus de fabrication d'électrodes extensibles 1D ;
- « Polymer-Enhanced Highly Stretchable Conductive Fiber Strain Sensor Used for Electronic Data Gloves », Chen Shuai ET AL, Advanced Materials Technologies, vol. 1, no. 7, 22 août 2016, page 1600136, décrit un capteur de contrainte avec fibres conductrices réalisé au moyen d'un tapis de nanofibres et de nanofils d'argent.

## RÉSUMÉ DE L'INVENTION

La présente invention se propose donc de résoudre ces inconvénients en fournissant une feuille ou un textile connecté c'est-à-dire muni de capteurs permettant la détection de plusieurs paramètres différents et capable d'être utilisé tant sur des petites surfaces que des grandes surfaces.

A cet effet, l'invention concerne une feuille selon l'une quelconque des revendications 1 ou 2.

L'invention concerne en outre un ensemble selon la revendication 15.

L'invention concerne également un dispositif de détection selon la revendication 16.

L'invention concerne en outre une structure selon la revendication 19.

L'invention concerne en outre un procédé de détection selon la revendication 21.

## DESCRIPTION DES FIGURES

D'autres particularités et avantages ressortiront clairement de la description qui en est faite ci-après, à titre indicatif et nullement limitatif, en référence aux dessins annexés, dans lesquels:

- les fig. 1, 2a, 2c et 3 sont des représentations schématiques de la feuille et du fil selon l'invention;
- la fig. 2b est un exemple de réalisation non couvert par les revendications d'un fil capteur sous forme de monofilament ;
- les fig. 4 à 10 sont des représentations schématiques de modes d'exécution de l'invention et de leurs variantes;
- la fig. 11 est une représentation schématique d'une variante du fil utilisé dans la feuille selon l'invention;
- les fig. 12 et 13 sont des représentations schématiques d'une connexion de plusieurs feuilles selon l'invention.

## DESCRIPTION DETAILLÉE DE L'INVENTION

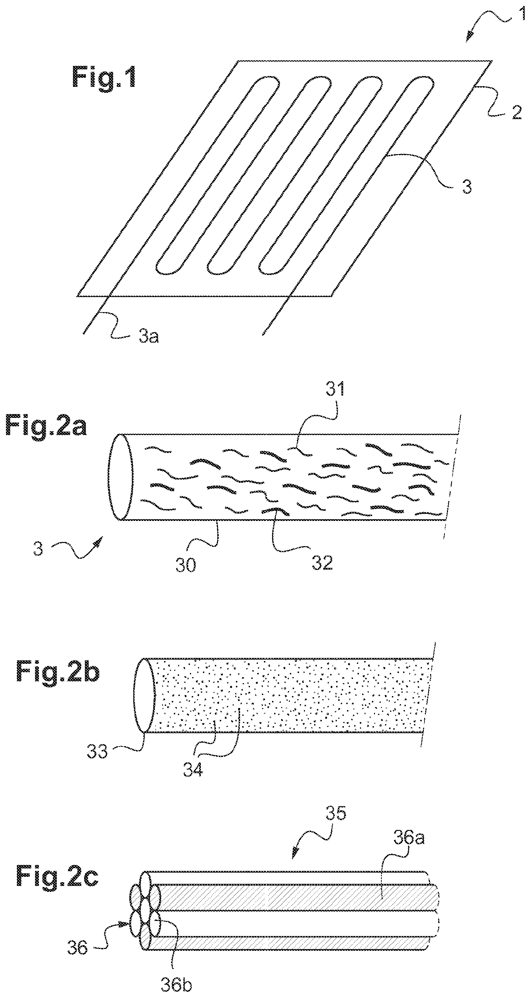

A la figure 1 est représentée une bande ou feuille connectée selon l'invention. Une telle bande ou feuille 1 est utilisée pour être appliquée/fixée sur des substrats tels que les plaques de plâtre, mortier, béton, dalle, brique, bois, carrelage, tuyau, la laine ou mousse d'isolation, ou encore des géotextiles. Cette feuille pourra ainsi être agencée sur les parois, mur, plafond ou sol d'un bâtiment. Cette feuille connectée est utilisée pour des applications du type système antieffraction ou détection de problèmes d'infiltration ou surveillance antisismique. Il sera également possible que la feuille se suffise à elle-même.

Cette bande 1 comprend un support 2. Ce support 2 est choisi isolant, de préférence souple et peut être un film plastique ou un textile (fibre de verre) ou un papier. Un textile est considéré comme un ensemble de fils ou de filaments arrangés entre eux de façon aléatoire ou non. Ce textile peut être tissé, non-tissé, tricoté, tressé. Le support 2 est également choisi pour être électriquement non conducteur.

A ce support 2 est associé au moins un fil 3 appelé encore fil capteur. Ce fil capteur comprend des composants isolants et conducteurs intimement liés et agencés pour permettre l'apparition de ponts conducteurs entre les composants conducteurs.

Dans un premier mode de réalisation visible à la figure 2a, le fil capteur 3 est un brin 30 constitué d'un filé de fibres conducteur composé de fibres isolantes polymériques 31 formant composants isolants et de fibres conductrices 32 formant composants conducteurs mélangés entre elles comme visible à la figure 2.

Dans un exemple de réalisation non couvert par les revendications et visible à la figure 2b, le fil capteur 3 est un monofilament 33 réalisé dans un matériau isolant formant composant isolant chargé de particules conductrices 34 formant composants conducteurs.

Dans un troisième mode de réalisation visible à la figure 2c, le fil capteur 3 est un brin 35 comprenant une multitude de filaments 36. Cette multitude de filaments comprend des filaments conducteurs 36a formant composants conducteurs et des filaments isolants 36b formant composants isolants.

Les fibres, filaments ou particules conductrices peuvent être de l'inox ou d'aluminium, du cuivre ou de l'argent, de l'or ou du nickel, du carbone, des nanotubes de carbone, du noir de carbone, du graphite, du graphène, des polymères organiques semi-conducteurs ou conducteurs comme le polypyrolle, le polyaniline, le polythiophène, le poly(p phénylène sulfide), le poly(p-phénylène vinylène, polyacétylènes, polyflurènes, polypyrènes, polyazulènes, polynaphtallènes, des fibres métallisées et/ou des fibres avec une enduction conductrice ou un ensimage conducteur.

Les fibres ou filaments isolants 31 peuvent être également de différents types : Polyester, Polypropylène, Polyéthylène, Polyamide, Aramide, Polyacide lactique, polyvinyle alcool, Polyacrylate, verre, quartz, polybenzoxazole, Polyméthylmétacrylate, polytetrafluoroethylene, polyimide, polyetherimide, polyuréthane, chlorofibre, élastanne, coton, laine, lin, chanvre, jute, sisal, coïr, bamboo, kenaf, ramie, soie, cellulose, viscose.

Ce fil capteur 3 est associé au support 2 c'est-à-dire qu'il est en contact avec le support 2. Le fil capteur 3 peut être co-tissé, co-tricoté ou co-tressé avec le support 2 sous forme de textile à des tensions bien spécifiques pour obtenir des sensibilités de détection optimales. Ce fil capteur 3 peut être également enduit et/ou lié chimiquement, mécaniquement, thermiquement et/ou hydrauliquement au support 2 sans ou avec un cycle de cuisson spécifique.

On comprend donc qu'une contrainte appliquée sur le support 2 est, de façon avantageuse, directement transmise audit fil capteur 3 qui réagit en conséquence.

Pour permettre la détection d'une contrainte extérieure sur la surface du support 2, ce fil 3 est agencé sur le support 2 pour s'étendre suivant une ou plusieurs directions pouvant être les dimensions (longueur et largeur) du support 2.

Un tel fil capteur 3 possède le fonctionnement suivant. La structure du fil capteur 3 est discontinue et comporte un fort taux de vide qui lui procure une certaine liberté de variation de diamètre sans pour autant changer l'organisation des fibres. Lorsqu'une force de traction est appliquée suivant l'axe du fil capteur 3, la diminution réversible du diamètre du fil capteur jusqu'à un certain point est observée. Cette limite correspond approximativement à celle de la zone élastique du fil à laquelle s'ajoutent les performances élastiques singulières des fibres/filaments isolants composant le fil capteur 3.

La quantité de fibres/filaments/particules conductrices 32 présente au sein du fil capteur 3 est en toute évidence un paramètre très important pour la conductivité du fil capteur. En effet, la conduction se fait par conductivité métallique, par « hopping » ou par effet tunnel et ceci nécessite le contact entre matériaux conducteurs ou une certaine proximité. La conduction dans un fil conducteur est donc liée au nombre de points de contact ou ponts entre les fibres/filaments/particules conductrices 32. Plus le taux massique des fibres/filaments/particules conductrices 32 est élevé, plus la probabilité de créer un pont conducteur est élevée. Parallèlement, le nombre de fibres/filaments/particules conductrices dans la section augmente avec l'augmentation du taux massique. Ainsi, le mécanisme de détection mécanique est basé sur le lien qui existe entre les stimulations mécaniques et la variation du nombre de ponts entre les fibres/filaments/particules conductrices 32.

Lorsque le support 2 muni du fil capteur 3 subit une contrainte mécanique comme un étirement, une sollicitation mécanique apparait et engendre des variations géométriques au sein du fil capteur 3. Dans le régime élastique, la variation du diamètre a pour effet le rapprochement des fibres conductrices 32 dans la section. Ceci semble avoir pour conséquence la création de nouveaux ponts conducteurs entre les fibres/filaments/particules conductrices 32 ou l'accentuation des ponts existants, accompagnée d'une augmentation proportionnelle de la conductivité. On comprend donc que l'augmentation du nombre de ponts conducteurs se fait à l'intérieur même du fil capteur 3, le fil capteur se suffisant à lui-même pour la détection d'une contrainte.

Le fait d'avoir un fil capteur 3 en partie réalisé en matériaux isolants permet d'avoir, en temps normal/au repos, une conductivité électrique la plus faible possible. Cette faible conductivité au repos permet, lors de l'application d'une contrainte, d'avoir une variation de la conductivité qui est plus nette car elle passe d'une valeur faible à une valeur haute. De plus, cette faible conductivité au repos permet d'avoir une amplitude de variation plus grande que pour un fil capteur ayant une conductivité au repos déjà importante. Cette plus grande amplitude permet de détecter plus facilement l'intensité de la contrainte.

Lorsque la contrainte appliquée à la feuille 1 selon l'invention augmente, le régime plastique relaye celui élastique. En fin de zone élastique, les fibres/filaments/particules conductrices 32 glissent les unes sur les autres et s'alignent puis s'éloignent les unes des autres lors de la zone plastique. En s'alignant, l'éventualité de créer un réseau conducteur continu augmente. Le fait d'augmenter encore la contrainte conduit à la dégradation graduelle du fil capteur 3 jusqu'à rupture. La conductivité suit la même tendance et diminue progressivement jusqu'à atteindre une conductivité nulle.

Par conséquent, il est possible de déterminer en partie l'importance du choc. Effectivement, si la variation de conductivité détectée se traduit, dans le temps, par une augmentation de la conductivité puis par une baisse de celle-ci jusqu'à atteindre une valeur nulle, alors il en sera déduit que la contrainte appliquée au substrat est telle qu'elle a entraîné une rupture dudit fil capteur 3.

De même, une conductivité détectée devenant quasi instantanément nulle signifie que la contrainte subie est due à un choc violent qui a sectionné net le fil capteur 3.

On comprend donc que le fil capteur 3 n'a pas besoin d'éléments intermédiaires pour voir sa conduction électrique se modifier suite à une contrainte sur le support 2. En particulier, il n'y a pas besoin d'éléments conducteurs supplémentaires agencés sur le support 2 pour modifier la conduction électrique dudit fil capteur 3.

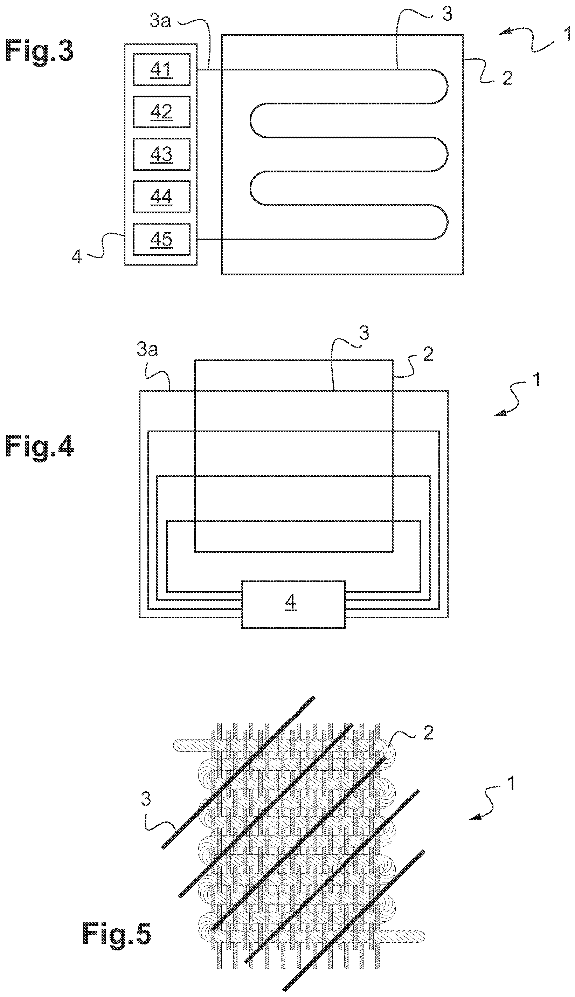

Les deux extrémités 3a du fil capteur 3 dont connectées à un circuit de détection 4 comme visible à la figure 3. Ce circuit de détection 4 est muni d'une unité de détection 41 pour détecter les variations de conductivité et fournir des valeurs représentatives de la conductivité, d'une unité de calcul 42 pour traiter les valeurs représentatives de la conductivité et fournir un signal représentatif, d'une mémoire 43 pour stocker les valeurs représentatives de la conductivité et un circuit de communication 44 pour transmettre le signal représentatif de façon filaire ou sans-fil, le tout étant alimenté par une unité d'alimentation 45 autonome ou se branchant sur le secteur. Le circuit de détection 4 est alors conçu pour envoyer un courant dans le fil et mesurer un paramètre électrique comme la conductivité ou la résistivité. Les valeurs mesurées sont sauvegardées et traitées pour qu'un signal représentatif du paramètre mesuré soit généré.

Selon l'invention, le fil capteur 3 peut être utilisé pour la détection d'autres paramètres.

Un second paramètre détecté par la bande/feuille 1 connectée selon l'invention est l'humidité. En effet, l'eau pénétrant dans le fil capteur 3 crée des ponts entre les fibres conductrices ce qui permet une augmentation significative de la conductivité électrique. Il devient possible de détecter ce paramètre avec le même fil capteur 3 que pour la détection de contrainte d'étirement.

Un troisième paramètre détectable pourra être la température. En effet, selon la loi de Nernst-Einstein qui permet de calculer la conductivité en fonction d'autres paramètres fondamentaux du matériau :

$$\sigma = \frac{D\,Z^2 e^2 C}{k_B T}$$

Où:

D est le coefficient de diffusion de l'espèce chargée considérée ;
Z est le nombre de charges portées par l'espèce ;
e est la charge élémentaire, soit $1,602 \times 10^{-19}$ C ;
C est la concentration molaire de l'espèce ;
$k_B$ est la constante de Boltzmann, soit environ $1,3806 \times 10^{-23}$ J·K$^{-1}$ ;
T est la température absolue, exprimée en kelvins.

On constate donc que cette conductivité dépend de la température de sorte que la conductivité diminue si la température augmente. Il devient ainsi possible de déterminer une variation de température subie par la feuille 1.

Pour opérer une détection d'une contrainte telle que l'élongation, la pression, la torsion, l'élévation de température et la présence de rayonnement, de gaz ou de liquide sur le support 2, plusieurs modes d'exécution sont prévus.

Dans un premier mode d'exécution visible à la figure 3, un seul fil capteur 3 est agencé sur le support 2. Dans ce cas, le fil capteur 3 comprend plusieurs portions rectilignes ou sensiblement rectilignes parallèles entre elles, deux portions parallèles l'une par rapport à l'autre étant reliées par une portion courbe afin que l'ensemble forme un seul fil capteur 3 continu. Ce mode d'exécution permet, avec un seul fil capteur 3, de détecter une contrainte comme un étirement ou la présence d'humidité avec un seul fil capteur 3 sur une grande surface.

Dans ce premier mode d'exécution, l'unité de détection 4 comprend donc deux bornes de connexion pour la connexion des deux extrémités 3a du fil capteur 3.

Dans un second mode d'exécution, plusieurs fils capteurs 3 sont agencés sur le support 2.

Selon une première solution à ce mode d'exécution visible à la figure 4, les fils 3 sont disposés sur le support 2 pour que chaque fil capteur 3 s'étende linéairement suivant une direction, lesdits fils capteurs 3 étant parallèles entre eux. Cette direction peut être parallèle à l'une des dimensions du support 2 ou angulairement décalée comme visible à la figure 5.

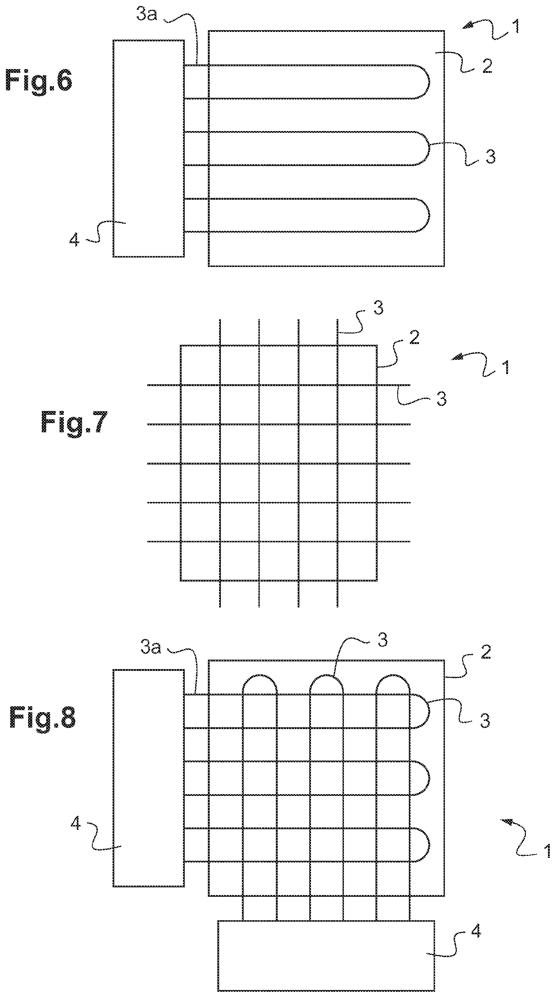

Selon une seconde solution à ce mode d'exécution visible à la figure 6, les fils capteurs 3 sont disposés sur le support 2 pour que chaque fil capteur 3 présente une forme composée de deux portions rectilignes ou sensiblement rectilignes parallèles reliées par une portion courbe. Les fils capteurs 3 sont ensuite agencés parallèlement les uns par rapport aux autres.

Ce second mode d'exécution permet de détecter une contrainte comme un étirement ou la présence d'humidité sur une grande surface avec plus de précisions. En effet, comme le support 2 est muni de plusieurs fils capteurs 3 indépendants les uns des autres, la présence d'une contrainte ou d'humidité en un point n'entraine pas une variation de la conduction électrique sur chacun des fils 3.

Par ailleurs, le fait d'avoir plusieurs fils capteurs 3 permet d'augmenter la fiabilité puisque la coupure d'un fil capteur 3 n'entraîne pas une impossibilité de détection pour l'ensemble du support 2 comme pour le premier mode d'exécution.

Enfin, comme la sensibilité de détection dépend en partie de la longueur du fil capteur 3, le second mode d'exécution permet d'avoir un circuit de détection 4 avec une sensibilité moins grande tout en gardant une détection aussi efficace.

Dans ce cas-là, il peut y avoir un circuit de détection 4 par fil capteur 3 ou un circuit de détection 4 pour l'ensemble des fils capteurs 3 comme représenté à la figure 4.

Dans le premier mode d'exécution et le second mode d'exécution, le ou les fils capteurs 3 seront agencés sur le support 2 pour permettre une couverture de détection sur au moins 60% de la surface du support 2, préférentiellement 75% et encore plus préférentiellement 90% de la surface dudit support.

Dans une variante du second mode d'exécution, les différents fils capteurs 3 sont agencés pour obtenir une précision de détection accrue. Pour cela, les fils capteurs 3 sont agencés pour former un maillage.

Dans le cas de la première solution dudit second mode d'exécution visible à la figure 7, le support 2 est muni de plusieurs séries de fils capteurs 3. Une première série comprend des fils capteurs 3 rectilignes, parallèles entre eux et disposés pour s'étendre selon une première direction. Une seconde série comprend des fils capteurs 3 rectilignes, parallèles entre eux et disposés pour s'étendre selon une seconde direction, la première direction et la seconde direction étant différentes. Les fils capteurs 3 de la première série et les fils capteurs 3 de la seconde série étant agencés sur le support 2 de sorte à ne pas être électriquement en contact. Le tout forme un quadrillage permettant de déterminer avec précision l'endroit où la contrainte détectée est située.

Dans le cas de la seconde solution dudit second mode d'exécution visible à la figure 8, l'agencement est réalisé de manière similaire que celui de la première solution. Une première série comprend des fils capteurs 3 rectilignes, parallèles entre eux et disposés pour s'étendre selon une première direction. Une seconde série comprend des fils capteurs 3 rectilignes, parallèles entre eux et disposés pour s'étendre selon une seconde direction, la première direction et la seconde direction étant différentes. Chaque fil capteur 3 présente une forme composée de deux portions rectilignes parallèles reliées par une portion courbe.

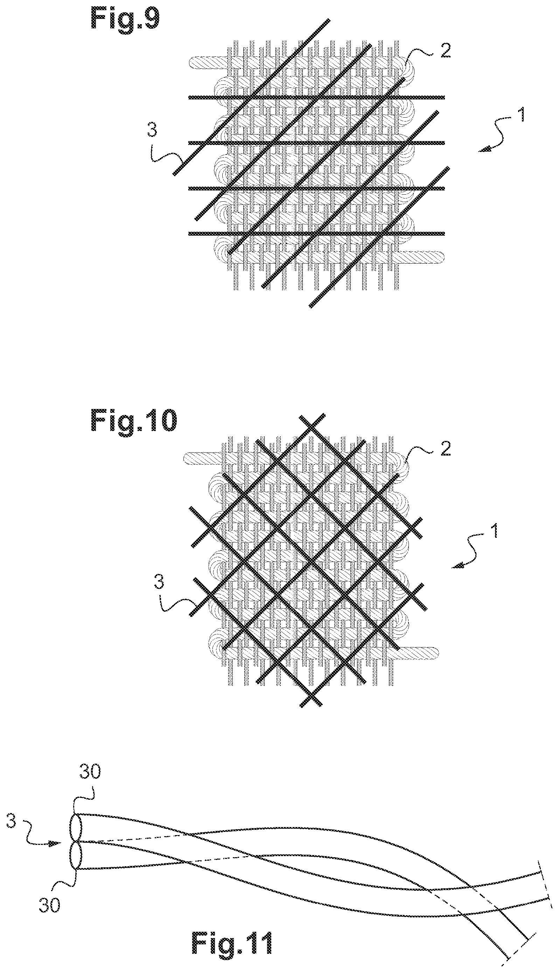

Pour cette variante du second mode d'exécution, la première direction et la seconde direction peuvent être perpendiculaire ou être sécantes avec un angle préférentiel de 45°. La première direction et la seconde direction peuvent être également être parallèles à la longueur/largeur du support ou être angulairement décalées par rapport à celles-ci comme visible aux figures 9 et 10. Cet angle pourra être choisi selon la forme du substrat. En effet, la surface du substrat sur lequel la bande/feuille 1 connectée est posée pourra ne pas être plane.

Dans cette variante du second mode d'exécution, il pourra être prévu d'avoir un circuit de détection 4 par fil 3 ou alors un circuit de détection 4 par série de fils 3 ou encore un circuit de détection 4 pour l'ensemble des fils 3.

Dans une variante de l'invention comme visible à la figure 11, le fil capteur 3 comprend plusieurs brins 30. Chaque brin 30 est constitué d'un filé de fibres conducteur composé de fibres isolantes polymériques 31 et de fibres conductrices métalliques 32 mélangés entre elles. Ces différents brins 30 sont torsadés afin de former un seul fil capteur 3. Le fil capteur 3 pourra comprendre un nombre de brins 30 allant de 2 à 5.

Le fait de multiplier le nombre de brins 30 permet d'une part d'avoir un fil capteur 3 plus résistant et plus fiable car une rupture partielle ou totale accidentelle d'un des brins n'occasionne plus de perturbations dans le fonctionnement du système.

De plus, ce renforcement du fil 3 a un impact sur le renforcement du support 2 voire du substrat porteur. Comme le fil 3 multibrins est agencé directement sur le support 2, il contribue à son renforcement mécanique. De la même façon, le renforcement mécanique du support 2 a un impact sur le substrat qui porte le tout.

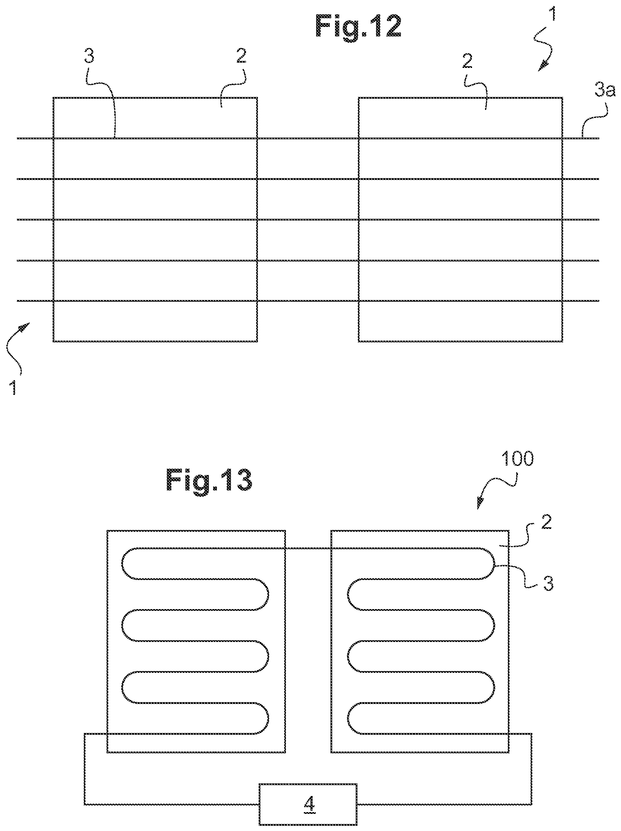

Dans une variante de l'invention visible aux figures 12 et 13, il pourra être prévu qu'une série de supports 2 puissent se connecter entre eux en série pour former un ensemble 100. Pour cela, les deux extrémités libres du ou des fils 3 de chaque support 2 pour la connexion au circuit de détection 4 sont utilisées. Une première extrémité d'un fil capteur 3 du premier support 2 est connectée au circuit de détection 4 alors que la seconde extrémité dudit fil capteur 3 est connectée à la première extrémité d'un fil capteur 3 du second support 2 et ainsi de suite. La seconde extrémité du fil capteur 3 du dernier support 2 de la série est connectée au circuit de détection 4.

Cette variante est aussi applicable pour des supports 2 auxquels sont associés plusieurs fils capteurs 3. Dans ce cas, chaque fil capteur 3 du support 2 est connecté à un fil capteur 3 du support 2 précédent et/ou suivant. Préférentiellement, les supports 2 sont configurés de la même manière c'est-à-dire que les fils capteurs 3 de ces supports 2 ont la même disposition. A ce titre, le fil capteur 3 d'un support 2 est connecté en série avec le fil capteur 3 du support suivant et/ou précédent ayant la même disposition permettant de simplifier la connexion en série des différents supports 2.

Dans cette variante, il pourra être prévu d'avoir un circuit de détection 4 par fil capteur 3 ou alors un circuit de détection 4 par série de fils capteurs 3 ou encore un circuit de détection 4 pour l'ensemble des fils capteurs 3.

Bien entendu, la présente invention ne se limite pas à l'exemple illustré mais est susceptible de diverses variantes et modifications qui apparaîtront à l'homme de l'art.

Par exemple, il pourra être prévu que le support sur lequel le fil capteur 3 est agencé soit rigide comme par exemple un support en verre. Cela permettrait d'équiper une vitre ou porte vitrée afin d'obtenir un dispositif anti-effraction voir un dispositif régulateur de température utilisant la variation de la conductivité électrique du fil en fonction de la température extérieur captée par le fil du vitrage pour commander un dispositif de climatisation/-chauffage.

De plus, il sera possible d'avoir un support 2 comprenant un fil capteur 3 rectiligne et un fil capteur 3 composé de deux portions rectilignes parallèles reliées par une portion courbe.

**Revendications**

1. Feuille (1) comprenant un support (2) en un matériau électriquement non conducteur et au moins un fil capteur (3) associé audit support, ledit fil capteur réagissant électriquement à une contrainte extérieure, ledit fil capteur comprenant des composants isolants et des composants conducteurs intimement liés et agencés pour permettre l'apparition de ponts conducteurs entre les composants conducteurs dudit fil capteur, et ledit fil capteur étant associé au support (2) de sorte qu'au moins une contrainte extérieure appliquée audit support soit directement transmise audit fil capteur pour provoquer une variation du nombre de ponts conducteurs et entrainer une réaction électrique, ledit fil comportant au moins un brin (30) formé d'un filé de fibres conducteur composé de fibres isolantes (31) et de fibres conductrices (32) mélangés entre elles, la feuille étant **caractérisée en ce que** le support (2) est composé de fils ou de filaments textiles arrangés entre eux de façon non-tissé.

2. Feuille (1) comprenant un support (2) en un matériau électriquement non conducteur et au moins un fil capteur (3) associé audit support, ledit fil capteur réagissant électriquement à une contrainte extérieure, ledit fil capteur comprenant des composants isolants et des composants conducteurs intimement liés et agencés pour permettre l'apparition de ponts conducteurs entre les composants conducteurs dudit fil capteur, et ledit fil capteur étant associé au support (2) de sorte qu'au moins une contrainte extérieure appliquée audit support soit directement transmise audit fil capteur pour provoquer une variation du nombre de ponts conducteurs et entrainer une réaction électrique, ladite feuille étant **caractérisée en ce que** ledit fil comporte une pluralité de filaments dont au moins deux sont conducteurs, le reste étant des filaments isolants.

3. Feuille selon la revendication 2 , **caractérisé en ce que** le support (2) est réalisé en un matériau plastique.

4. Feuille selon la revendication 2 , **caractérisé en ce que** le support (2) est composé de fils ou de filaments textiles arrangés entre eux de façon aléatoire ou non pouvant être tissé, non-tissé, tricoté, tressé.

5. Feuille selon la revendication 4, **caractérisé en ce que** le support (2) est composé de fibres de verres.

**6.** Feuille selon la revendication 2 , **caractérisé en ce que** le support (2) est réalisé en papier.

**7.** Feuille selon l'une des revendications précédentes, **caractérisé en ce que** ledit fil capteur comporte une pluralité de brins, de préférence entre deux et cinq.

**8.** Feuille selon l'une des revendications précédentes, **caractérisé en ce qu'**elle comprend un unique fil capteur (3).

**9.** Feuille selon la revendication précédente, **caractérisé en ce que** ledit fil capteur présente une forme comprenant une pluralité de portions rectilignes parallèles entre elles, chaque portion comprenant deux extrémités lui permettant d'être reliée aux portions précédentes et suivantes par une portion courbe.

**10.** Feuille selon l'une des revendications 1 à 7, **caractérisé en ce qu'**elle comprend une pluralité de fils capteurs.

**11.** Feuille selon la revendication précédente, **caractérisé en ce que** chaque fil capteur (3) présente une forme rectiligne.

**12.** Feuille selon la revendication 10, **caractérisé en ce que** ledit chaque fil capteur présente une forme comprenant deux portions rectilignes parallèles entre elles et reliées par une portion courbe.

**13.** Feuille selon l'une des revendications 10 à 12, **caractérisé en ce que** la pluralité de fils s'étend selon une seule direction.

**14.** Feuille selon l'une des revendications 10 à 12, **caractérisé en ce que** la pluralité de fils capteurs s'étend selon au moins deux directions sécantes, préférentiellement perpendiculaire, pour former un maillage.

**15.** Ensemble (100) comprenant une pluralité de feuilles (1) selon l'une des revendications précédentes, **caractérisé en ce que** deux feuilles (2) adjacentes sont connectées en série.

**16.** Dispositif de détection comprenant la feuille (1) ou l'ensemble (100) selon l'une des revendications précédentes, **caractérisé en ce qu'**il comprend en outre un circuit de détection (4) connecté au(x)dit(s) fil(s) capteur (3) pour détecter les réactions électrique lors de contraintes mécaniques appliquées au(x)dit(s) support(s) (2) et transmises directement au(x)dit(s) fil(s).

**17.** Dispositif de détection selon la revendication 16, **caractérisé en ce qu'**il comprend un circuit de détection (4) pour connecter l'ensemble des fils capteurs.

**18.** Dispositif de détection selon la revendication 16, **caractérisé en ce qu'**il comprend, pour chaque fil capteur (3), un circuit de détection.

**19.** Structure comprenant un substrat et le dispositif de détection selon l'une des revendications 16 à 18, le dispositif de détection étant disposé sur le substrat.

**20.** Structure selon la revendication 19, **caractérisé en ce que** ledit substrat est choisi dans la liste comprenant : plaque de plâtre, mortier, béton, brique, carrelage, tuyau, la laine ou mousse d'isolation, le bois.

**21.** Procédé de détection d'une contrainte extérieure sur un dispositif de détection selon l'une des revendications 16 à 18, ledit procédé comprenant les étapes suivantes :

- se munir du dispositif de détection ;
- envoyer, séquentiellement ou de façon continue, un courant dans le fil capteur (3) par le circuit de détection (4);
- mesurer un paramètre électrique, de préférence la conductivité, et sauvegarder ces valeurs mesurées ;
- traiter ces valeurs dans le circuit de détection pour générer un signal représentatif du paramètre électrique.

**Patentansprüche**

**1.** Folie (1), umfassend einen Träger (2) aus elektrisch nicht leitfähigem Material und mindestens einen Sensordraht (3), der dem Träger zugeordnet ist, wobei der Sensordraht auf eine äußere Beanspruchung elektrisch reagiert, der Sensordraht umfassend isolierende und leitfähige Komponenten, die eng miteinander verbunden und zum Ermöglichen der Entstehung von leitfähigen Brücken zwischen den leitfähigen Komponenten des Sensordrahts angeordnet sind, und wobei der Sensordraht dem Träger (2) zugeordnet ist, sodass mindestens eine äußere Beanspruchung, die auf den Träger ausgeübt wird, zum Hervorrufen einer Veränderung der Anzahl von leitfähigen Brücken und Bewirken einer elektrischen Reaktion direkt auf den Sensordraht übertragen wird, wobei der Draht mindestens einen Einzeldraht (30) aufweist, der aus einem leitfähigen Fasergarn ausgebildet ist, das aus miteinander vermischten isolierenden Fasern (31) und leitfähigen Fasern (32) besteht, wobei die Folie **dadurch gekennzeichnet ist, dass** der Träger (2) aus textilen Drähten oder Filamenten besteht, die auf nicht gewebte Weise zusammengefügt sind.

**2.** Folie (1), umfassend einen Träger (2) aus elektrisch nicht leitfähigem Material und mindestens einen Sensordraht (3), der dem Träger zugeordnet ist, wobei der Sensordraht auf eine äußere Beanspruchung elektrisch reagiert, der Sensordraht umfassend isolierende und leitfähige Komponenten, die eng miteinander verbunden und zum Ermöglichen der Entstehung von leitfähigen Brücken zwischen den leitfähigen Komponenten des Sensordrahts angeordnet sind, und der Sensordraht dem Träger (2) zugeordnet ist, sodass mindestens eine äußere Beanspruchung, die auf den Träger ausgeübt wird, zum Hervorrufen einer Änderung der Anzahl der leitfähigen Brücken und Bewirken einer elektrischen Reaktion direkt auf den Sensordraht übertragen wird, wobei die Folie **dadurch gekennzeichnet ist, dass** der Draht eine Vielzahl von Filamenten aufweist, von denen mindestens zwei leitfähig sind, die übrigen isolierende Filamente sind.

**3.** Folie nach Anspruch 2, **dadurch gekennzeichnet, dass** der Träger (2) aus einem Kunststoffmaterial hergestellt ist.

**4.** Folie nach Anspruch 2, **dadurch gekennzeichnet, dass** der Träger (2) aus textilen Drähten oder Filamenten hergestellt ist, die zufällig oder nicht zufällig zusammengefügt sind, gewebt, nicht gewebt, gestrickt oder geflochten sein kann.

**5.** Folie nach Anspruch 4, **dadurch gekennzeichnet, dass** der Träger (2) aus Glasfasern besteht.

**6.** Folie nach Anspruch 2, **dadurch gekennzeichnet, dass** der Träger (2) aus Papier hergestellt ist.

**7.** Folie nach einem der vorstehenden Ansprüche, **dadurch gekennzeichnet, dass** der Sensordraht eine Vielzahl von Einzeldrähten, vorzugsweise zwischen zwei und fünf, aufweist.

**8.** Folie nach einem der vorstehenden Ansprüche, **dadurch gekennzeichnet, dass** sie einen einzelnen Sensordraht (3) umfasst.

**9.** Folie nach dem vorstehenden Anspruch, **dadurch gekennzeichnet, dass** der Sensordraht eine Form vorweist, umfassend eine Vielzahl von geradlinigen Abschnitten, die parallel zueinander verlaufen, jeder Abschnitt umfassend zwei Enden, die es ihm ermöglichen, durch einen gekrümmten Abschnitt mit den vorstehenden und den folgenden Abschnitten verbunden zu werden.

**10.** Folie nach einem der Ansprüche 1 bis 7, **dadurch gekennzeichnet, dass** sie eine Vielzahl von Sensordrähten umfasst.

**11.** Folie nach dem vorstehenden Anspruch, **dadurch gekennzeichnet, dass** jeder Sensordraht (3) eine geradlinige Form vorweist.

**12.** Folie nach Anspruch 10, **dadurch gekennzeichnet, dass** jeder Sensordraht eine Form vorweist, umfassend zwei geradlinige Abschnitte, die parallel zueinander verlaufen und durch einen gekrümmten Abschnitt verbunden sind.

**13.** Folie nach einem der Ansprüche 10 bis 12, **dadurch gekennzeichnet, dass** sich die Vielzahl von Drähten in eine einzige Richtung erstreckt.

**14.** Folie nach einem der Ansprüche 10 bis 12, **dadurch gekennzeichnet, dass** sich die Vielzahl von Sensordrähten in mindestens zwei sich kreuzende Richtungen, vorzugsweise senkrecht, zum Ausbilden eines Gewirkes erstreckt.

**15.** Satz (100), umfassend eine Vielzahl von Folien (1) nach einem der vorstehenden Ansprüche, **dadurch gekennzeichnet, dass** zwei benachbarte Folien (2) in Reihe angeschlossen sind.

**16.** Erfassungsvorrichtung, umfassend die Folie (1) oder den Satz (100) nach einem der vorstehenden Ansprüche, **dadurch gekennzeichnet, dass** sie ferner eine Erfassungsschaltung (4), die an den/die Sensordraht/-drähte (3) angeschlossen ist, zum Erfassen der elektrischen Reaktionen während mechanischer Beanspruchungen umfasst, die auf den/die Träger (2) ausgeübt und direkt auf den/die Draht/Drähte übertragen werden.

**17.** Erfassungsvorrichtung nach Anspruch 16, **dadurch gekennzeichnet, dass** sie eine Erfassungsschaltung (4) zum Anschließen des Satzes von Sensordrähten umfasst.

**18.** Erfassungsvorrichtung nach Anspruch 16, **dadurch gekennzeichnet, dass** sie für jeden Sensordraht (3) eine Erfassungsschaltung umfasst.

**19.** Struktur umfassend ein Substrat und die Erfassungsvorrichtung nach einem der Ansprüche 16 bis 18, wobei sich die Erfassungsvorrichtung auf dem Substrat befindet.

**20.** Struktur nach Anspruch 19, **dadurch gekennzeichnet, dass** das Substrat aus der Liste ausgewählt ist, umfassend: Gipsplatte, Mörtel, Beton, Ziegel, Fliesen, Rohr, Wolle oder Isolierschaum, Holz.

**21.** Verfahren zum Erfassen einer äußeren Beanspruchung auf einer Erfassungsvorrichtung nach einem der Ansprüche 16 bis 18, das Verfahren umfassend die folgenden Schritte:

- Ausrüsten mit der Erfassungsvorrichtung;
- Senden, sequenziell oder kontinuierlich, eines Stroms in dem Sensordraht (3) durch die Erfassungsschaltung (4);
- Messen eines elektrischen Parameters, vorzugsweise der Leitfähigkeit, und Speichern dieser Messwerte;
- Verarbeiten dieser Werte in der Erfassungsschaltung zum Erzeugen eines Signals, das den elektrischen Parameter darstellt.

**Claims**

1. Sheet (1) comprising a carrier (2) made of an electrically non-conductive material and at least one sensor wire (3) associated with said carrier, said sensor wire reacting electrically to an exterior stress, said wire comprises insulating components and conductive components that are intimately associated and arranged to allow conductive bridges to appear between the conductive components of said wire, and said wire being associated with the carrier (2) so that at least one exterior stress applied to said carrier is directly transmitted to said sensor wire in order to provoke a variation in the number of conductive bridges and cause an electrical reaction, said wire including at least one strand (30) formed from a spun yarn of conductive fibres, which yarn is composed of intermixed insulating fibres (31) and conductive fibres (32), the sheet being **characterized in that** the carrier (2) is composed of textile filaments or wires arranged with respect to one other in a nonwoven way.

2. Sheet (1) comprising a carrier (2) made of an electrically non-conductive material and at least one sensor wire (3) associated with said carrier, said sensor wire reacting electrically to an exterior stress, said wire comprises insulating components and conductive components that are intimately associated and arranged to allow conductive bridges to appear between the conductive components of said wire, and said wire being associated with the carrier (2) so that at least one exterior stress applied to said carrier is directly transmitted to said sensor wire in order to provoke a variation in the number of conductive bridges and cause an electrical reaction, said sheet being **characterized in that** said wire includes a plurality of filaments at least two of which are conductive, the rest being insulating filaments.

3. Sheet according to claim 2, **characterized in that** the carrier (2) is made of a plastic material.

4. Sheet according to claim 2, **characterized in that** the carrier (2) is composed of textile filaments or wires that are or are not randomly arranged with respect to one other, the carrier being woven, nonwoven, knitted or tressed.

5. Sheet according to Claim 4, **characterized in that** the carrier (2) is composed of glass fibres.

6. Sheet according to claim 2, **characterized in that** the carrier (2) is made of paper.

7. Sheet according to one of the preceding claims, **characterized in that** said sensor wire includes a plurality of strands and preferably between two and five strands.

8. Sheet according to one of the preceding claims, **characterized in that** it comprises a single sensor wire (3).

9. Sheet according to the preceding claim, **characterized in that** said sensor wire has a shape comprising a plurality of rectilinear segments that are parallel to one another, each segment comprising two ends allowing it to be connected to the preceding and following segments by a curved segment.

10. Sheet according to one of Claims 1 to 7, **characterized in that** it comprises a plurality of sensor wires.

11. Sheet according to the preceding claim, **characterized in that** each sensor wire (3) has a rectilinear shape.

12. Sheet according to Claim 10, **characterized in that** said each sensor wire has a shape comprising two rectilinear segments that are parallel to each other and connected by a curved segment.

13. Sheet according to one of Claims 10 to 12, **characterized in that** the plurality of wires extends in a single direction.

14. Sheet according to one of Claims 10 to 12, **characterized in that** the plurality of sensor wires extends in at least two secant directions that are preferably perpendicular, in order to form a mesh.

15. Assembly (100) comprising a plurality of sheets (1) according to one of the preceding claims, **characterized in that** two adjacent sheets (2) are connected in series.

16. Detecting device comprising the sheet (1) or the assembly (100) according to one of the preceding claims, **characterized in that** it furthermore comprises a detecting circuit (4) that is connected to said sensor wire(s) (3) in order to detect electrical reactions during mechanical stresses applied to said carrier(s) (2) and transmitted directly to said wire(s).

**17.** Detecting device according to Claim 16, **characterized in that** it comprises one detecting circuit (4) for connection to all of the sensor wires.

**18.** Detecting device according to Claim 16, **characterized in that** it comprises one detecting circuit for each sensor wire (3).

**19.** Structure comprising a substrate and the detecting device according to one of Claims 16 to 18, the detecting device being placed on the substrate.

**20.** Structure according to Claim 19, **characterized in that** said substrate is chosen from the list comprising: plasterboard, mortar, concrete, brick, tiling, piping, insulating foam or wool, and wood.

**21.** Method for detecting an exterior stress on a detecting device according to one of Claims 16 to 18, said method comprising the following steps:

- providing the detecting device;
- sending, sequentially or continuously, a current to the sensor wire (3) via the detecting circuit (4);
- measuring an electrical parameter, preferably conductivity, and saving these measured values;
- processing these values in the detecting circuit to generate a signal representative of the electrical parameter.

Fig.1

Fig.2a

Fig.2b

Fig.2c

Fig.3

Fig.4

Fig.5

Fig.6

Fig.7

Fig.8

**Fig.9**

**Fig.10**

**Fig.11**

# Fig.12

# Fig.13

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4400684 A
- US 2006254366 A
- WO 0175778 A
- US 2008170982 A
- US 4794365 A
- BE 1017472

**Littérature non-brevet citée dans la description**

- **LEE JAEHONG et al.** Recent Advances in 1D Stretchable Electrodes and Devices for Textile and Wearable Electronics: Materials, Fabrications, and Applications. *Advanced Materials*, 01 January 2019, vol. 32 (5), 1-28
- **CHEN SHUAI et al.** Polymer-Enhanced Highly Stretchable Conductive Fiber Strain Sensor Used for Electronic Data Gloves. *Advanced Materials Technologies*, 22 August 2016, vol. 1 (7), 1600136